Europäisches Patentamt

European Patent Office

Office européen des brevets

(11) Publication number: 0 391 495
A1

# EUROPEAN PATENT APPLICATION

(21) Application number: 90200824.2

(51) Int. Cl.⁵: A45D 20/04

(22) Date of filing: 06.04.90

(30) Priority: 07.04.89 JP 41584/89 U

(43) Date of publication of application:
10.10.90 Bulletin 90/41

(84) Designated Contracting States:
CH DE FR GB IT LI NL

(71) Applicant: YOSHIHARA & CO. LTD.
17-4, Shibuya-1-chome Shibuya-ku
Tokyo(JP)

(72) Inventor: Yoshihara, Kenjiro
17-4, Shibuya 1-chome
Shibuya-ku, Tokyo(JP)

(74) Representative: Smulders, Theodorus A.H.J.,
Ir. et al
Vereenigde Octrooibureaux Nieuwe Parklaan
107
NL-2587 BP 's-Gravenhage(NL)

(54) Hot air pulse generator.

(57) A hot air pulse generator comprises a hot air generating device including a heater and an air blower (10), having a blowout hole (5) on a side opposite to the hot air generating device, defining a passageway, which leads to the hot air generating device, a damper (14) arranged across the passageway (11) of the housing, which converges hot air, as high-speed drift, toward one side of the passageway (11), a butterfly valve (15) rotated by the hot air, as drift, which is generated by the damper (14).

FIG. I

## BACKGROUND OF THE INVENTION

The present invention relates to a hot air pulse generator. More particularly, the invention relates to a hot air pulse generator, utilizing a hot air generating apparatus such as a hair drier, in particular, or the like, or an apparatus which combines an air blower with a heat source of a far infrared radiation heat treatment apparatus and the like.

Conventionally, hot air generating apparatuses, such as, for example a hair drier, blow out a uniform continuous flow of hot air of a fixed quantity and wind pressure, from a blowout hole. Such a hair drier is so constructed as to achieve only its inherent functions: blow-drying, setting hair, etc. The apparatus with the heat source of the far infrared radiation heat treatment apparatus is also so constructed as to achieve only its inherent functions.

It is an object of the present invention to provide a hot air pulse generator, which, in addition to its inherent functions, massages to the head, or other parts of the body needing to be messaged by means of hot air blown out in a pulse-like manner, the hot air being obtained from the hot air generating apparatus or the apparatus which combines the air blower with the heat source of the far infrared radiation heat treatment apparatus.

## SUMMARY OF THE INVENTION

A hot air pulse generator comprises hot air generating means including a heater and an air blower, a housing having a blowout hole on a side opposite to the hot air generating means, said housing defining a passageway leading to the hot air generating means, a damper arranged across the passageway of the housing for converging hot air, as high-speed drift, toward one side of the passageway, a butterfly valve rotated by the hot air, as drift, which is generated by the damper.

Hot air, as a high-speed drift caused by a damper, strikes against a butterfly valve and is stopped from blowing out of a blowout hole, when the butterfly valve is closed. The hot air provides the butterfly valve with a turning force. When the butterfly valve is rotated by the turning force, a blowout hole opens, allowing the hot air to blow out of the hole. When the butterfly valve is rotated again, the valve is closed again, thus stopping the hot air from being blown out. At the same time, the valve is provided with the turning force. By repeating the steps described above, the hot air blows out, not in a uniform flow with a fixed quantity and wind pressure, but in an intermittent pulse-like flow, which is alternately strong and weak.

Other features will become apparent from the following Detailed Description of the Preferred Embodiment when read together with reference to the accompanying drawings.

## BRIEF DESCRIPTION OF THE DRAWINGS

FIG. 1 is a section view showing a hot air pulse generator, of a hair drier, of an embodiment according to the present invention;

FIG. 2 is a front view illustrating the hot air pulse generator.

## DETAILED DESCRIPTION OF THE PREFERRED EMBODIMENTS

An embodiment of the present invention will now be described with reference to the accompanying drawings. In this invention, the embodiment is applied to a hair drier as a hot air pulse generator.

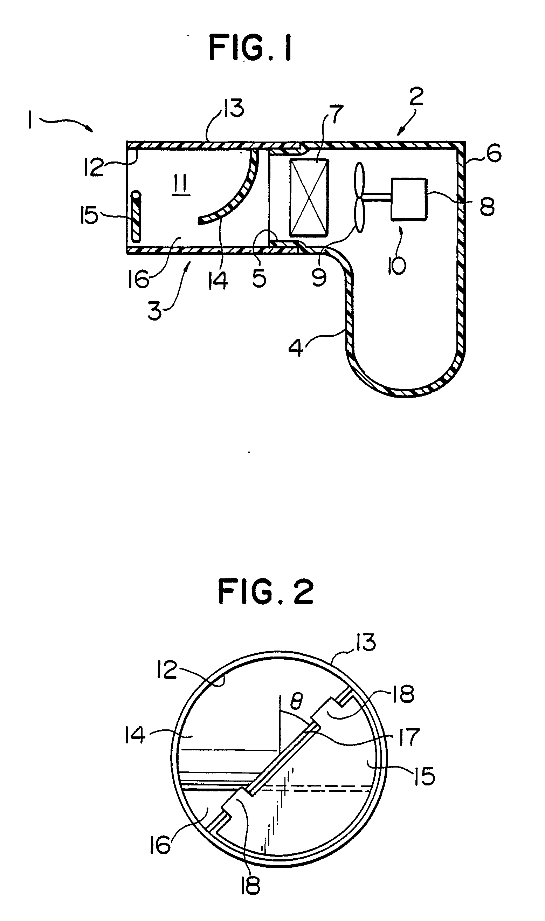

As shown in FIG. 1, the hot air pulse generator 1 of a hair drier comprises a hot air generating portion 2 and a pulse generating portion 3. The hot air generating portion 2 includes a housing 6, which has a handle 4 and a primary blowout hole 5, just like an ordinary hair drier. Within the housing 6 are arranged an electric heater 7, and an air blower 10, including an electric motor 8 and a fan 9. The electric heater 7 is a nichrome wire heater of a general type. The pulse generating portion 3 is attached to the primary blowout hole 5 of the housing 6 and defines a passageway 11, which connects the pulse generating portion 3 and the primary blowout hole 5. A cylindrical housing 13, having a secondary blowout hole 12, is so arranged as to oppose the hot air generating portion 2. A damper 14 is disposed across a passageway 11 of the cylindrical housing 13. Hot air flowing through the passageway 11 is converged as high-speed drift toward one side of the passageway 11. A butterfly valve 15, preferably a single-wing butterfly valve, is provided at the secondary blowout hole 12 of the cylindrical housing 13. The butterfly valve 15 is rotated by means of the hot air, as drift, generated by the damper 14.

In order to converge the hot air flowing through the passageway 11 toward one side of the passageway 11, the damper 14 should not be arranged perpendicular to the passageway 11 so as to stop the flow of the hot air, but rather, as shown in FIG. 1, the damper 14 may as well be formed as a gentle curve which curves toward a damper opening 16 so as to guide the hot air smoothly toward one side of the passageway 11. Further, as shown in FIG. 2, the damper 14 is desirably so constructed that the cut-off area by the damper 14 covers more than half the area of the opening of the passageway 11.

As shown in FIG. 2, the butterfly valve 15 is arranged across the secondary blowout hole 12, and has fixing portions 18 with inserting apertures. A spindle 17 fixed to the cylindrical housing 13 by screws or the like (not shown) runs through the inserting apertures. The butterfly valve 15 is rotatably attached to the spindle 17 at the fixing portions 18. The shape of the butterfly valve 15 is semicircular, which has a slightly smaller diameter than the inside diameter of the cylindrical housing 13. The spindle 17 should not be attached to extend in the direction in which the damper opening 16 is positioned. That is, the spindle 17 should not be attached so as to be parallel to the vertical direction in FIG. 2, but the spindle 17 is desirably attached at an inclination angle of $\theta$ relative to the vertical direction. The butterfly valve 15 is correspondingly attached at the angle of $\theta$.

The housing 6, the cylindrical housing 13, the damper 14 and the butterfly valve 15 may be molded by a highly heat-resistant material, such as polycarbonate. The damper 14 may be molded as an integral part of the cylindrical housing 13, or recessed into the housing, by using another material.

In this embodiment as constructed above, the hot air, generated by the air blower 10 and the electric heater 7, flows out of the primary blowout hole 5, and is converged toward one side of the passageway 11 because of the damper 14. Thus, the hot air, as high-speed drift, flows out of the damper opening 16. The hot air, as high-speed drift, strikes against the butterfly valve 15 and is stopped from blowing out of the blowout hole 12, when the valve is closed, as shown in FIG. 2. At the same time, the hot air provides the butterfly valve 15 with a turning force. When the butterfly valve is rotated by the turning force, the blowout hole opens, thus allowing the hot air to flow out of the blowout hole 12 smoothly. When the butterfly valve 15 is rotated again, the valve is closed again, thus stopping the hot air from being blown out. At the same time, the butterfly valve 15 is provided with the turning force. By repeating the steps described above, the hot air blows out, not in a uniform flow with a fixed quantity and wind pressure, but in an intermittent pulse-like flow, which is alternately strong and weak.

In this embodiment, the conditions under which the hot air pulse is generated depend upon the ratio of the cut-off area of the damper 14 to the opening area of the passageway 11 and upon an attaching angle $\theta$ of the spindle 17 in relation to the opened direction of the damper opening 16.

That is, if the ratio of the cut-off area by the damper 14 decreases, the effect of the hot air pulse decreases because of the following correlative reasons: a slow flow velocity caused by less converged drift, and a fact that the single-wing butterfly valve is provided with less turning force. On the contrary, if the ratio increases, although the butterfly valve is provided with a strong turning force, the single-wing butterfly valve is rotated too fast, thus generating almost a continuous flow of the hot air. As a result, the hair drier gives an uncomfortable feeling, when using it.

Further, if the attaching angle $\theta$ of the spindle 17 in relation to the opened direction of the damper opening 16 approaches an angle of $0°$, the area where hot air, as drift, strikes against, increases when the single-wing butterfly valve is closed again by another $180°$ rotation after the valve is closed, as shown in FIG. 2. When the valve is thus closed, the single-wing butterfly valve is rotated slowly, because a collision of the hot air against the damper functions as a force which weakens the turning force. Thus, the effect of the hot air pulse decreases. On the contrary, if the attaching angle $\theta$ of the spindle 17 in relation to the opened direction of the damper opening 16 approaches an angle of $90°$, an opposite phenomenon to the above occurs, i.e., the single-wing butterfly valve is rotated too fast, thereby the hot air generates almost a continuous flow.

According to a test result, the optimum pulse-like hot air was obtained under the following conditions:
the ratio of the cut-off area by the damper 14 to the area of opening of the passageway 11 was 50 - 75%, and the attaching angle $\theta$ of the spindle 17 in relation to the opened direction of the damper opening 16 was approximately an angle of $45°$.

According to the present invention, while using a hair drier, the pulse-like blown hot air massages the head and improves the circulation of the blood around the hair roots.

In this embodiment, although the single-wing butterfly valve 15 is used, a double-wing butterfly valve is also acceptable.

Moreover, where the butterfly valve is a single-wing or double-wing type instead of the semicircular plate butterfly valve 15 described above, a bowl-like shaped butterfly valve may be applied. A butterfly valve with a weight attached may also be applied to obtain, when required, more stable rotation of the valve. In the case of the butterfly with a weight, it is inferred that the desirable attaching angle of the spindle 17 fluctuates from the above-mentioned angle.

Furthermore, in this embodiment, although the housing 6 and the cylindrical housing 13 are composed as separate portions, they may be composed together as an integral part.

An embodiment of the present invention has been described with particular reference to a hot air pulse generator of a hair drier. The present invention hitherto described is not limited to the above-described hot air pulse generator, but may also be applied to other apparatuses with heat sources, such as a far infrared radiation heat treatment apparatus.

It is possible according to the present invention to provide a product, which, in addition to the inherent functions of a hot air pulse generator of a hair drier, or an apparatus with a heat source of a far infrared radiation heat treatment apparatus, massages the head or other parts of the body needing to be massaged.

The invention has been described in detail with particular reference to the preferred embodiment thereof, but it will be understood that variations and modifications of the invention can be effected within the spirit and scope of the invention.

## Claims

1. A hot air pulse generator comprising:
hot air generating means including a heater and an air blower;
a housing having a blowout hole on a side opposite to said hot air generating device, said housing defining a passageway leading to said hot air generating means;
a damper arranged across said passageway of said housing for converging hot air, as high-speed drift, toward one side of said passageway;
a butterfly valve rotated by said hot air, as drift, which is generated by said damper.

2. A hot air pulse generator according to claim 1, wherein said butterfly valve is a single-wing butterfly valve.

3. A hot air pulse generator according to claim 2, wherein said single-wing butterfly valve is semicircular, and has a slightly smaller diameter than the inside diameter of said housing.

4. A hot air pulse generator according to claim 1, wherein said damper is formed as a gentle curve which curves toward said blowout hole of said housing.

5. A hot air pulse generator according to claim 1, wherein said damper has the cut-off area thereof, which covers more than half the opening area of said housing.

6. A hot air pulse generator according to claim 5, wherein the cut-off area covers substantially 50 to 75% of said opening area.

7. A hot air pulse generator according to claim 1, wherein said butterfly valve is rotatably attached to a spindle, which is fixed across said blowout hole of said housing, and said spindle is attached at a predetermined angle in relation to a line, which runs through the center of said hot air, as drift, and the center of said blowout hole.

8. A hot air pulse generator according to claim 7, wherein said predetermined angle is substantially 45°.

4

# FIG. 1

# FIG. 2

European Patent Office

**EUROPEAN SEARCH REPORT**

Application number

| | DOCUMENTS CONSIDERED TO BE RELEVANT | | EP 90200824.2 |

| Category | Citation of document with indication, where appropriate, of relevant passages | Relevant to claim | CLASSIFICATION OF THE APPLICATION (Int Cl ⁵) |
|---|---|---|---|
| A | <u>US - A - 4 210 162</u><br>(DREYER et al.)<br>* Totality *<br>-- | 1 | A 45 D 20/04 |
| A | <u>DE - A1 - 2 708 117</u><br>(WILHELM KRUK NACHF.)<br>* Totality *<br>-- | 1 | |
| A | <u>CH - A - 452 809</u><br>(GRAMESPACHER)<br>* Totality *<br>---- | 1 | |

| | | | TECHNICAL FIELDS SEARCHED (Int Cl ⁵) |
|---|---|---|---|
| | | | A 45 D |

The present search report has been drawn up for all claims

| Place of search | Date of completion of the search | Examiner |
|---|---|---|
| VIENNA | 18-06-1990 | NETZER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82